Europäisches Patentamt

**European Patent Office**

**Office européen des brevets**

(11) Publication number: **0 062 261**
A1

(12) **EUROPEAN PATENT APPLICATION**

(21) Application number: 82102578.0

(22) Date of filing: 26.03.82

(51) Int. Cl.³: **C 07 C 17/00**, C 07 C 25/02

(30) Priority: 31.03.81 JP 48147/81

(43) Date of publication of application: 13.10.82
Bulletin 82/41

(84) Designated Contracting States: DE FR GB IT

(71) Applicant: **TORAY INDUSTRIES, INC., 2, Nihonbashi-Muromachi 2-chome Chuo-ku, Tokyo 103 (JP)**

(72) Inventor: **Tada, Kuniyuki, 2-3-5 Tsu-Nishi, Kamakura-shi Kanagawa-ken (JP)**
Inventor: **Minomiya, Eiichi, 1111 Tebiro, Kamakura-shi Kanagawa-ken (JP)**
Inventor: **Iwayama, Kazuyoshi, 2-1-17 Tsu-Nishi, Kamakura-shi Kanagawa-ken (JP)**
Inventor: **Inoue, Takehisa, 2-24-11 Noge, Setagaya-ku Tokyo (JP)**

(74) Representative: **DRes. Kador & Klunker, Corneliusstrasse 15, D-8000 München 5 (DE)**

(54) **Process for isomerizing a halogenated toluene.**

(57) Provided is a process for isomerizing a halogenated toluene using acid zeolite as a catalyst in the presence of benzene, alkylbenzene or halogenated benzene as a diluent.

EP 0 062 261 A1

Process for Isomerizing a Halogenated Toluene

Background of the Invention

This invention relates to a process for the catalytic isomerization of halogenated toluenes.

The term "halogenated toluenes" herein involves toluenes monohalogen-substituted on their nuclei, more particularly, isomers of o-halogenated toluene, m-halogenated toluene and p-halogenated toluene. The term "halogen" involves chlorine, bromine, iodine and fluorine.

Generally, halogenated toluenes are obtained by nuclear substitution reaction of toluene with halogens. This halogenation reaction exhibits a strong ortho-para orientation. Accordingly, if a m-isomer of the halogenated toluene is required, it is necessary to isomerize o- or p-isomer.

The proportion of the respective isomers of a halogenated toluene demanded is different from proportion of them formed by the halogenation in many cases. Therefore, an isomerization method has an important technical significance also for the effective utilization of the halogenated toluene.

As an isomerization reaction, there have been known, for example, a method wherein aluminium chloride or the like is used as a catalyst as disclosed in G.A. Olah, J. Org. Chem. 27, 3464 (1964) and a method wherein HF-BF$_3$ in used as a catalyst as disclosed in Japanese Patent Publication No. 11809/1971. However, these known isomerization methods are not preferred as industrial isomerization methods, since they have problems that the catalytic activity is insufficient, a long reaction time is required, a large amount of the catalyst is necessitated, the reaction device is corroded and the separation of the reaction product from the catalyst is difficult.

After investigations made for the purpose of overcoming these defects of the conventional methods and establishing an industrially advantageous process for isomerizing halogenated toluenes, the inventors found previously that acid zeolites have remarkable effects.

Generally, in an industrial process comprising the catalytic isomerization reaction carried out in the presence
of a solid acid catalyst such as an acid zeolite, it is
important that the catalyst has a sufficient activity and
selectivity and also that the catalytic activity is maintained for a long period of time or, in other words,
deterioration of the catalytic activity with time must be
only slight. The catalytic isomerization method wherein
a solid acid catalyst such as an acid zeolite is used
has been employed widely on an industrial scale in the
isomerization of parafinic hydrocarbons, olefinic- hydrocar-
bons and alkylaromatic hydrocarbons. However, in these
isomerization processes, the catalytic activity is generally deteriorated with reaction time and, therefore, the
activity must be maintained by elevating the reaction
temperature as the catalytic activity is deteriorated-
with time and when the reaction temperature has reached
the highest temperature allowed in the isomerization
reaction, the catalyst is regenerated or discharged. Thus,
in case the deterioration of catalytic activity with time
is serious, the reaction condition (temperature) must be
altered or the regeneration must be effected frequently,
whereby the operation becomes troublesome. Further, cost
of the regeneration or the catalyst becomes high. Thus,
the above-mentioned processes are not yet satisfactory
industrial processes.

The deterioration of catalytic activity with time in
the isomerization reaction carried out in the presence of
the above-mentioned solid acid catalyst is caused by the
following reasons:

(1) deposition of carbonaceous materials on the catalyst
surface,

(2) denaturation of the active site due to thermal history,
etc., and

(3) poisoning by catalyst poisons.

Among the above-mentioned reasons, the deposition
of carbonaceous materials on the catalyst surface is
responsible for the deterioration to the highest degree
in general.

In the catalytic isomerization process wherein a solid
acid catalyst is used, the reaction is carried out generally in a gas phase in the presence of hydrogen so as to
prevent the deterioration of catalytic activity with time
due to the deposition of carbonaceous materials on the
catalyst surface or the reaction is carried out in a liquid phase so as to wash away the carbonaceous precursors
from the catalyst surface by the reaction liquid itself.
However, it has been found that in the conventional processes for the catalytic isomerization of halogenated
toluenes carried out in the presence of an acid zeolite
catalyst, the deterioration of the isomerization activity with time is serious and that such processes are unsatisfactory industrially.

An object of the present invention is to overcome this defect of the conventional method.

## Summary of the Invention

It has been found by the present inventors that the deterioration of isomerization activity with time can remarkably be prevented by using a specific compound as a diluent. The present invention has been attained on the basis of this finding.

The present invention provides a process for isomerizing a halogenated toluene by contacting the halogenated toluene with an acid zeolite, characterized in that the isomerization is carried out in the presence of one or more compounds selected from the group consisting of benzene, alkylbenzenes and halogenated benzenes, a weight ratio of the compound(s) to the halogenated toluene being 1/20 to 20/1.

Brief Description of Drawings

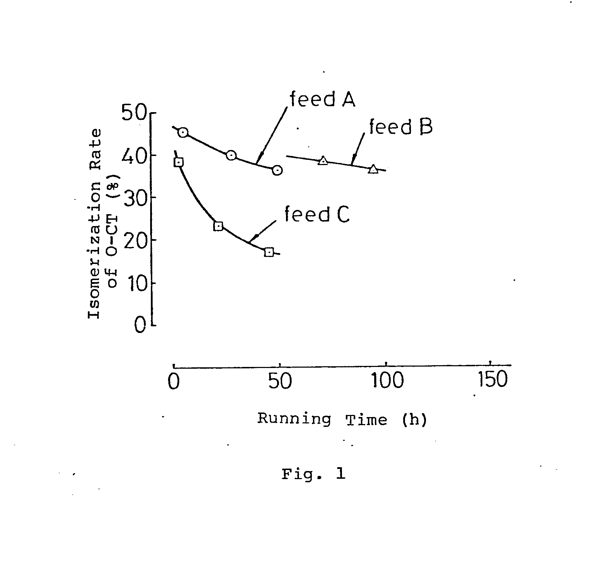

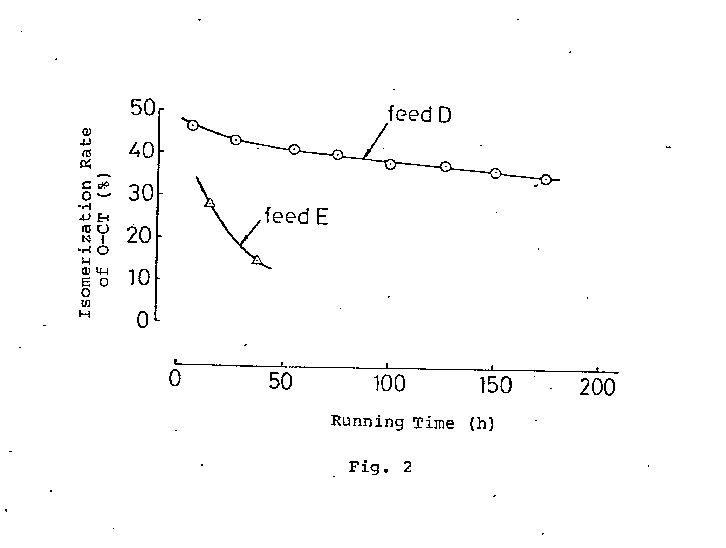

Figs. 1 and 2 show the results obtained in Examples 1 and 2 and Comparative Examples 1 and 2. In these figures, feeds A, B and D contain a diluent and feeds C and E are free of any diluent.

Detailed Description of the Invention

In the isomerization process of the present invention, it is an indispensable condition that at least one compound selected from the group consisting of benzene, alkylbenzenes and halogenated benzenes is used as a diluent in the isomerization reaction. The alkylbenzenes used in the present invention are those having 1-4 alkyl groups having 1-2 carbon atoms , and when two or more alkyl groups are contained, they may be the same as or different from one another. As the alkylbenzenes, there may be mentioned, for example, 'toluene, ethylbenzene,

xylene, diethylbenzene, ethyltoluene and trimethylbenzene.
The halogenated benzenes are those having 1-4 halogen
atoms, the halogen being fluorine, chlorine, bromine or
iodine, and when two or more halogen atoms are contained,
they may be the same as or different from one another.
As the halogenated benzenes, there may be mentioned, for
example, fluorobenzene, chlorobenzene, bromobenzene,
iodobenzene, dichlorobenzene, dibromobenzene and bromochlorobenzene.

In industrial processes, it is preferable that diluents used in the reaction satisfy the following conditions:
(1)  boiling point of the diluent is not close to those
of the reactants and products, so that the diluent can
easily be separated from the latter, and
(2)  side reactions such as decomposition are not easily
caused under the isomerization reaction conditions and
by-products which cannot be separated easily from the
reactants and products are not formed.

However, boiling points of halogenated toluenes vary
depending on the halogen contained therein and, in addition,
they have various isomerization reactivities.  Therefore,
the optimum reaction conditions are various.  Thus, the
diluent should be selected depending on variety of the
halogenated toluenes.  For example, in case chlorotoluene
is used as the halogenated toluene, preferred diluents are benzene, toluene and chlorobenzene. Among them, benzene
is particularly preferred.

The diluents may be used either alone or as a mixture
of two or more of them. In either case, the diluent should
be used in the isomerization in·such an amount that a
weight ratio of the diluent to the halogenated toluene
is 1/20 to 20/1. If the weight ratio of diluent to halogenated toluene is less than 1/20, the effect of preventing
the deterioration of catalytic activity with time is insufficient. On the other hand, even if said weight ratio is
increased above 20/1, the effect of preventing the deterioration of catalytic activity with time is not substantially
increased any more and rather the increase in quantity of
the liquid in the isomerization process increases the
energy consumption unfavorably. A preferred weight ratio
of the diluent to the halogenated toluene is 1/5 to 5/1.

As for the zeolites used in the present invention,
any natural and synthetic zeolites having such a pore
diameter that the halogenated toluene is diffusible thereinto under the reaction conditions may be used. No parti-
cular limitation is made also on the crystalline structures
of the zeolites. However, if an atomic ratio of Si to Al
in the zeolite is too low, the acid strength is low and
the isomerization activity is also low unfavorably, since
the zeolite used in the isomerization reaction according to the present invention acts as a solid acid catalyst.
Preferred zeolites are those having an atomic ratio of
Si to Al of at least 2. As preferred zeolites, there
may be mentioned, for example, mordenite and ZSM-5 type
zeolites having main cavities with openings constituted
by a 10-membered oxygen ring. Among them, ZSM-5 type
zeolites are particularly preferred.

The term "ZSM-5 type zeolites" herein involves
zeolite ZSM-5 and zeolites considered to be analogous to
it. The composition and process for the production of
zeolite ZSM-5 are disclosed in the specification of
U.S. Patent No. 3,894,106 and its crystalline structure
is described in "Nature" 271, 30 March, 437 (1978). As
such zeolites, there may be mentioned, in addition to
said ZSM-5, for example, ZSM-8 disclosed in the specification of British Patent No. 1,334,243, ZSM-11 disclosed
in the specification of Japanese Patent Publication No.
23280/1978, ZSM-21 disclosed in the specification of
U.S. Patent No. 4,001,346, ZSM-35 disclosed in the specification of Japanese Patent Laid-Open No. 144,500/1978,
zeolite Z (zeta) 1 disclosed in the specification of Japanese Patent Laid-Open No. 67299/1976, zeolite Z (zeta)
3 disclosed in the specification of Japanese Patent Laid-
Open No. 67,298/1976 and zeolite disclosed in the specification of Japanese Patent Application No. 189719/81. Among these ZSM-5 type zeolites, the last one is the most preferable. It is a novel crystallin aluminosilicate zeolite having the X-ray diffraction pattern of Table 1 and can be prepared by a following method. An aqueous reaction mixture consisting of silica source, an alumina source, an alkali source and an organic compound having at least one carboxlyl group is prepared in such a manner that it is within some particular composition range. Then, the aqueous reaction mixture is reacted until crystals of zeolite are formed.

Table 1    X-ray diffraction pattern

| $d(\overset{\circ}{A})$ | $100 \ I/I_0$ |
|---|---|
| 11.2 ± 0.2 | S |
| 10.2 ± 0.2 | S |
| 9.8 ± 0.2 | M |
| 6.37 ± 0.1 | W |
| 6.00 ± 0.1 | W |
| 5.71 ± 0.1 | W |
| 5.58 ± 0.1 | W |
| 4.37 ± 0.08 | W |
| 4.27 ± 0.08 | W |
| 3.86 ± 0.08 | VS |
| 3.82 ± 0.02 | VS |
| 3.75 ± 0.08 | S |
| 3.72 ± 0.08 | S |
| 3.66 ± 0.05 | M |
| 3.00 ± 0.05 | M |
| 2.00 ± 0.05 | W |

In the above table the relative strength ($100\ I/I_0$) is expressed as follows: VS = very strong, S = strong, M = medium strength, W = weak.

In the process of the present invention, the zeolites are used in acid form. As well known, acid zeolites contain a proton or polyvalent cation having a valence of at least 2 such as a rare earth ion. They are obtained by ion-exchanging at least part of monovalent alkali metal ion such as sodium with proton or polyvalent cation or by ion-exchanging the same with an ammonium cation followed by converting into a proton by calcination. A ZSM-5 type zeolite generally contains an organic nitroten-containing cation and so is calcined to decompose the organic nitrogen-containing cation and to give proton, whereby an acid zeolite ·is obtained. Any of those zeolites having an acidity required for the isomerization reaction of the halogenated toluene may be used in the presen invention irrespective of the variety or amount of the cation.

The zeolites are used generally in the agglomerated form in the isomerization process of the present invention. The aggromeration method is not particularly limited and known methods such as rolling, extrusion and compression methods may by employed. If necessary, a binder such as alumina sol and caly may be incorporated therewith in the agglomerating operation. The ion exchange may be effected either before or after the agglomeration of the zeolite. The zeolite articles thus formed are activated by calcining them generally at 300-700°C to form the catalyst.

In the isomerization process of the present invention, at least one halogenated toluene selected from the group consisting of o-, m- and p-halogenated toluenes having no thermodynamically equilibrium composition is catalytically isomerized in the presence of the acid zeolite prepared as above in the coexistence of at least one compound selected from the group consisting of benzene, alkylbenzenes and halogenated benzenes.

The reaction can be carried out according to known isomerization processes in the coexistence of the above-mentioned diluent(s). Satisfactory results can be obtained in either a gas phase or a liquid phase. In the gas phase reaction, it is more preferred that the reaction is carried out in the coexistence of also hydrogen gas. Any of fixed bed, moving bed and fluidized bed systems may be employed. The fixed bed flow system is preferred for the reaction from the viewpoint of easiness of the operation, since the catalytic activity is maintained for a long period of time in this system according to the process of the present invention. The reaction temperature is generally about 150-500°C, particularly 200-400°C. The reaction pressure is not particularly limited, though, as a matter of course, it should be controlled so as to maintain the reaction system in a liquid state depending on varieties of the halogenated toluene and diluent.

Weight hourly space velocity (WHSV) based on a halogenated toluene is 0.05-30 $hr^{-1}$, preferably 0.1-20 $hr^{-1}$.

According to the process of the present invention, selectivity in the isomerization reaction is also improved in addition to the effect of preventing the deterioration of isomerization activity of the halogenated toluene with time. Transalkylation or transhalogenation reaction occurs in the isomerization reaction of the halogenated toluene like the isomerization reaction of an alkylaromatic hydrocarbon such as xylene. However, these side reactions can be controlled by the addition of the above-mentioned diluent in the isomerization reaction.

The following examples will further illustrate the present invention.

Example 1

Zeolite ZSM-5 powder having a $SiO_2/Al_2O_3$ molar ratio of 45.5 was synthesized according to a method disclosed in the specification of Japanese Patent Laid-Open No. 54598/1975. 15 wt.% (as $Al_2O_3$) of alumina sol was added to the ZSM-5 powder. The whole was kneaded, extruded to obtain 14-24 mesh pellet and calcined at 500°C for 2 h in air. The pellet of ZSM-5 was ion-exchanged 5 times using 10 wt.% aqueous ammonium chloride solution (solid/liquid ratio: 2.0 1/kg, at about 90°C), washed thoroughly with water, dried at 120°C for 15 h and calcined at 500°C for 2 h in air to obtain an acid ZSM-5 catalyst.

The isomerization reaction of o-chlorotoluene (O-CT) was carried out using the acid ZSM-5 catalyst in a gas phase in a fixed bed flow reactor while a mixture of chlorobenzene (CB) and O-CT (feed A) and a mixture of benzene (Bz) and O-CT (feed B) were fed by turns by means of a changing switch.

The reaction conditions were as follows:

Compositions of the liquid feeds:

 Feed A: CB/O-CT = 1/1 wt/wt

 Feed B: Bz/O-CT = 1/1 wt/wt

$H_2$/feed: 5/1 mol/mol

WHSV (O-CT): 2.0 $h^{-1}$

Reaction temperature: 290°C

Reaction pressure: 20 $kg/cm^2$-G

A change in isomerization rate of O-CT with time is shown in Fig. 1. It will be understood that as compared with Comparative Example 1 wherein O-CT alone was used as the feed, the deterioration with time was reduced remarkably in the isomerization carried out in the coexistence of CB or Bz. The O-CT isomerization rate was calculated from the following equation:

O-CT isomerization rate (%)

$$= \frac{\text{(O-CT/CT in the liquid feed)} - \text{(O-CT/CT in the product liquid)}}{\text{(O-CT/CT in the liquid feed)}} \times 100$$

Comparative Example 1

The isomerization reaction of O-CT was carried out under the same reaction conditions as in Example 1 except that O-CT alone was used as the feed (feed C). A change in isomerization rate of O-CT with time is shown in Fig. 1.

Example 2

The isomerization reaction of O-CT was carried out in a liquid phase in the coexistence of CB (feed D) in the presence of the same catalyst in the same fixed bed flow reactor as in Example 1.

The reaction conditions were as follows:

Composition of the liquid feed: Feed D CB/O-CT=1/2 wt/wt WHSV (O-CT): $1.0 \text{ h}^{-1}$

Reaction temperature: 275°C

Reaction pressure: $30 \text{ kg/cm}^2\text{-G}$

A change in isomerization rate of O-CT with time is shown in Fig. 2. As compared with Comparative Example 2, the deterioration of O-CT isomerizing activity with time is remarkably reduced in the coexistence of CB, while the deterioration is serious in the absence of the diluent also in the liquid phase reaction.

Comparative Example 2

The isomerization reaction of O-CT was carried out under the same reaction conditions as in Example 2 except that O-CT alone was used as the feed (feed E). A change in isomerization rate of O-CT with time is shown in Fig. 2.

Example 3

Zeolite ZSM-5 powder having a $SiO_2/Al_2O_3$ molar ratio of 24.5 was prepared according to a method disclosed in the specification of Japanese Patent Laid-Open No. 54598/ 1975. 15 wt.% (as $Al_2O_3$) of alumina sol was added to the ZSM-5 powder. The whole was kneaded, extruded to obtain 14-24 mesh pellet and calcined at 500°C for 2 h in air. The pellet of ZSM-5 was ion-exchanged 5 times using 10 wt.% aqueous ammonium chloride solution (solid/ liquid ratio: 2.0 l/kg, at about 90°C), washed thoroughly with water, dried at 120°C for 15 h and calcined at 550°C for 2 h in air to obtain an acid ZSM-5 catalyst.

The isomerization reaction of O-CT was carried out using the acid ZSM-5 catalyst in a liquid phase in a fixed bed flow reactor in the coexistence of Bz.

The reaction conditions were as follows:

Composition of the liquid feed: Bz/O-CT = 1/2 wt/wt WHSV (O-CT): 1.0 $h^{-1}$

Reaction temperature: 265°C

Reaction pressure: 35 $kg/cm^2$-G

A change in isomerization rate of O-CT is shown in the following table:

| Running time (h) | O-CT isomerization rate (%) |
|---|---|
| 24 | 46.1 |
| 48 | 45.8 |
| 68.5 | 45.2 |
| 140 | 44.2 |

Example 4

The isomerization reaction of O-CT was carried out in the coexistence of toluene (Tol) in a liquid phase using the same catalyst and reaction device as in Example 3.

The reaction conditions were as follows:

Composition of the liquid feed: Tol/O-CT = 1/2 wt/wt WHSV (O-CT): 1.0 $h^{-1}$

Reaction temperature: 270°C

Reaction pressure: 35 $kg/cm^2$-G.

A change in isomerization rate of O-CT is shown in the following table:

| Running time (h) | O-CT isomerization rate (%) |
|---|---|
| 24 | 38.6 |
| 51 | 37.4 |
| 69.5 | 36.9 |

Example 5

According to a method disclosed in the specification of Japanese Patent Application No. 189719/81, aqueous reaction mixture consisting of 66.0 g. of silicic acid powder, 17.47 g. of a sodium aluminate solution, 9.22 g. of a solid sodium hydroxide, 12.5 g. of tartaric acid and 344.2 g. of water was reacted at 160°C for 72 h with stirring to obtain zeolite powder having a $SiO_2/Al_2O_3$ molar ratio of 25.2. 15 wt % (as $Al_2O_3$) of alumina sol was added to thus obtained zeolite powder. The whole was kneaded, extruded to obtain 14-24 mesh pellet and calcined at 500°C for 2 h in air. The pellet of the zeolite was ion-exchanged 5 times using 10 wt % aqueous ammonium chloride solution (solid/liquid ratio: 2.0 1/kg, at about 90°C), washed throughly with water, dried at 120°C for 15 h and calcined at 550°C for 2 h in air to obtain an acid zeolite catalyst.

Using thus obtained acid zeolite catalyst, the isomerization reaction of O-CT was carried out in a liquid phase in a fixed bed flow reactor in the coexistence of Bz.

The reaction conditions were as follows:

Composition of the liquid feed: Bz/O-CT = 1/2 wt/wt WHSV (O-CT): 1.0 $h^{-1}$

Reaction temperature: 265°C

Reaction pressure: 35 $kg/cm^2$-G.

A change in isomerization rate of O-CT is shown in the following table:

| Running time (h) | O-CT isomerization rate (%) |
|---|---|
| 29 | 40.9 |
| 70.5 | 40.8 |
| 119.5 | 40.5 |
| 171.5 | 39.3 |

Claims:

1.   A process for isomerizing a halogenated toluene by contacting the halogenated toluene with an acid zeolite, characterized in that the isomerization is carried out in the presence of one or more compounds selected from the group consisting of benzene, alkylbenzenes and halogenated benzenes, a weight ratio of the compound(s) to the halogenated toluene being 1/20 to 20/1.

2.   The process as defined in claim 1, wherein said alkylbenzenes have 1-4 alkyl group(s) having 1-2 carbon atom(s).

3.   The process as defined in claim 1, wherein said halogenated benzenes have 1-4 halogen atom(s), the halogen being fluorine, chlorine, bromine or iodine.

4.   The process as defined in claim 1, wherein said acid zeolite has an atomic ratio of silicon to aluminum of at least 2.

5.   The process as defined in claim 1, wherein said acid zeolite has main cavities with openings constituted by a 10-membered oxygen ring.

6. A process for isomerizing a halogenated toluene by contacting the halogenated toluene with an acid zeolite, characterized in that the isomeriztion is carried out in a gas phase or a liquid phase in the presence of one or more compounds selected from the group consisting of benzenes, alkylbenzenes and halogenated benzenes, a weight ratio of the compound(s) to the halogenated toluene being 1/20 to 20/1.

7. A process for isomerizing a halogenated toluene by contacting the halogenated toluene with an acid zeolite, characterized in that the isomerization is carried out in a gas phase in the presence of hydrogen gas and one or more compounds selected from the group consisting of benzene, alkylbenzenes and halogenated benzenes, a weight ratio of the compound(s) to the halogenated toluene being 1/20 to 20/1.

8. A process for isomerizing a chlorotoluene by contacting a chlorotoluene with an acid zeolite, characterized in that the isomerization is carried out in the presence of one or more compounds selected from the group consisting of benzene, toluene and chlorobenzene, a weight ratio of the compound(s) to the chlorotoluene being 1/20 to 20/1.

9. The process as defined in claim 8, wherein said compound is benzene.

1/2

Fig. 1

Fig. 2

## DOCUMENTS CONSIDERED TO BE RELEVANT

| Category | Citation of document with indication, where appropriate, of relevant passages | Relevant to claim |
|---|---|---|
| A,P | EP - A1 - 0 046 665 (TORAY INDUSTRIES)<br><br>* claims 1 to 8 *<br><br>---- | 1-5 |

**CLASSIFICATION OF THE APPLICATION (Int. Cl. 3)**

C 07 C 17/00

C 07 C 25/02

**TECHNICAL FIELDS SEARCHED (Int.Cl. 3)**

C 07 C 17/00

C 07 C 25/00

C 07 C 25/02

The present search report has been drawn up for all claims

| Place of search | Date of completion of the search | Examiner |
|---|---|---|
| Berlin | 25-06-1982 | KNAACK |

EPO Form 1503.1  06.78